# EUROPEAN PATENT APPLICATION

(11) **EP 1 844 793 A1**
(43) Date of publication of application: **17.10.2007**
(21) Application number: 06425266.1
(22) Date of filing: 13.04.2006
(51) Int. Cl.: A61K 47/48

(54) **Cyclic AMP binding molecule as vehicle for gene therapy**

(71) Applicant: D'Angelo, Sante, 64014 Martinsicuro (Teramo) (IT)
(72) Inventor: D'Angelo, Sante, 64014 Martinsicuro (Teramo) (IT)
(74) Representative: Faggioni, Carlo Maria

(57) **Abstract**

A vehicle for gene therapy is described consisting of a molecule capable of binding with cyclic AMP, such as a steroid hormone, in particular a sexual hormone, or DDT.

Preferably, said vehicle is used as a solution for parenteral administration.

## Description

The present invention refers to a vehicle for gene therapy which is not dangerous for the bodies it is applied to.

Gene therapy is the transfer of genetic material for the purpose of preventing or treating a disease. In the case of genetic disorders, in which there is a defective or missing gene, it essentially consists in the transfer of the working gene version to the host's body, so as to fix the defect.

As known, gene therapy provides the introduction of the fixing gene sequence, which is obtained from a viral sequence according to a random search method, into the cell nucleus which encodes the protein of interest.

As can be easily understood, this method is not suited to its purpose, due to the great danger entailed in the introduction of a viral sequence into a body. As a matter of fact, viruses can be identified as intracellular parasites, and hence, even though they have had their damaging features removed, they might nevertheless bind with any genetic sequence of human DNA and become part of a wider gene sequence, which may prove harmful or lethal to the body.

Moreover, if in the lesser complex bodies the input of a given gene sequence into the cellular nucleus is likely to allow to carry through fixing operations within the DNA, in the case of more complex bodies the introduction of genetic material into a random location may change the effect of said material according to the gene location, by which the alleles of a gene may be transcribed or not transcribed according to the location thereof in the nucleus, or it can suppress or un-suppress gene functions which are important, if not vital, to the body.

As a result, considering that 98% of the human genetic inheritance is deactivated after having been used during embryogenesis, inactive gene sequences may easily wake up and teratogenic, mutagenic or carcinogenic events may consequently occur. The above risk affects not only the individual, but also his or her offspring, whom he or she may produce following a gene therapy.

A further drawback of the prior art concerns the need to use costly and invasive genetic engineering techniques in order to introduce the genes into the DNA.

It is therefore an object of the present invention to supply a vehicle for gene therapy which overcomes these drawbacks, in particular which is able to introduce the genetic material into the same location of the chromosome which encodes said genetic material.

Said objects are achieved through a vehicle for gene therapy, characterised in that it consists of a molecule capable of binding with cyclic AMP.

The invention will now be described in greater detail with reference to preferred embodiments thereof.

It is known in the art to introduce the gene into the exact chromosome location which encodes the said gene by using DNA fixing means. Once the DNA undergoes lesions, it immediately provides to repair itself, severing the damaged sequence through an endonuclease, rebuilding it through a polymerase and destroying the defective portion through an exonuclease. Subsequently, the rebuilt portion is joined through a ligase.

This same technique can hence be applied, with the due expedients, to introduce the gene of interest in the required position. It becomes of course necessary to know exactly the biological mechanisms associated with this process at the time it is activated and the mechanisms causing the gene to operate inside the nucleus must consequently be known.

For this to be possible it is necessary to proceed through three different steps:
- introducing the genetic material in the nucleus,
- using the DNA repairing means to introduce the genes, and
- arranging the genes in question on the chromosome.

In order to be able to complete the first step, it is necessary to use as a vehicle molecules capable of crossing the entire cell and of entering the nucleus. For example, steroid and sexual hormones are capable of binding to cyclic AMP, once they have entered the cell, and through this vector, they are capable of arriving at the nucleus, also due to their fat-solubility.

A variety of other molecules may also be used for the purpose, especially synthetic ones. This type of molecule, in particular, may be useful for gene therapy in situations which exhibit complications. For example, the DDT molecule, which is toxic per se, may be used to kill a tumoral cell.

In order to introduce these substances into the body, an out-patient parenteral administration is sufficient, which may be inoculated in a single step if the dosage is sufficient to introduce the gene into all the cells.

As a matter of fact, the parenteral technique allows spreading of the therapeutic molecules according to the classic method used with drugs. This means that gene therapy with the vehicle according to the present invention will follow the conventional ways of pharmacokinetics and pharmacodynamics and not the costly and risky ways of genetic engineering.

Gene administration through the parenterally-introduced hormone transport sequence spreads the gene across the whole body, transported by the bloodstream. As a result, the gene in question, bound to the transport sequences, is carried into the cell and subsequently into the cell nucleus, into which it is introduced.

The vector for transportation of the sequence of hormones and of other agents engineered to contain the gene in the nucleus is cyclic AMP. It is therefore necessary that this be found in the hormone transportation sequence of the receptor site for cyclic AMP.

Operationally, the hormone is modified so that the gene of interest replaces a suitably-removed portion of the original molecule. The complex hormone-cyclic AMP-gene thus has access to the cell nucleus. Here, the hormone-cyclic AMP complex is removed from the cell in the known manner, whereas the gene is in the nucleus.

The second step provides to introduce the gene into the DNA: this might be a problem, since mammals' DNA is doublestranded. However, once the gene has been inserted, said gene lies opposite its complementary gene on the opposite strand; the latter will not be identical, and they will hence not bind in a suitable manner. At this point the DNA self-mending mechanism activates itself, in a fully spontaneous way, correcting altered bases.

However, it is necessary to use a clever device to prevent the self-mending mechanism from re-establishing the initial, pre-therapy condition. As a matter of fact, it is sufficient to introduce the operational gene sequence, i.e. the one on which the self-mending mechanism is based, to fix the defect. Thereby a pair of healthy gene alleles will be obtained.

Finally, the third step concerns gene arrangement on the chromosome. This is the step in which the DNA self-adjusting mechanisms are not sufficient, since they are not capable of carrying out insertion of a gene from the outside. Now, since the complex hormone-cyclic AMP-gene has been introduced into the chosen strand precisely so that it may be the operational gene acting as a mold, the DNA-mending means will consequently be able to use this strand to change the gene of the other strand. In the meantime, a branched abnormal complex develops on the individual chromosome strand.

This abnormality will lead to the DNA removing anything which may prevent it from preserving its strand structure, and hence to removing what has been introduced through the removal-enzyme, DNA polymerase I (or Kornberg's enzyme).

It is hence necessary to act on said enzyme. As a matter of fact, considering the fact that it consists of two sub-units with exonuclease activity, the larger fragment contains polymerase and exonuclease activity in the direction from 3' to 5', whereas the smaller one contains exonuclease activity from 5' to 3'. Typically, synthesis direction is along the smaller fragment; therefore, it will be sufficient to orientate the chain of the gene to be inserted along the direction 5'-3', cutting the DNA chain which was to remain closed. This - far from being a problem - aids introduction of the gene which must first be released, so that DNA ligase - implicated by Kornberg's enzyme - may bind the gene to the chain of normal DNA.

In order to ensure a favourable outcome of the process, it is advisable to arrange multiple genes on the location, the majority of which will then be removed, because once the binding terminal has been taken up by the individual gene on the strand of the corresponding chromosome, said excess genes will no longer be useful.

In some individuals, Kornberg's enzyme may work in the opposite direction; in this case it will be sufficient to introduce in the solution the DNA polymerase fragment which orientates the enzyme position on the strand in an opposite direction, so as to re-establish the desired direction.

In order to avoid useless traumas to the patient, it can be established if this method is to be applied by performing a simple *in vitro* test on blood cells.

Once the gene introduction procedure is completed, it is necessary to activate such gene, but this too is relatively simple: it is sufficient to cause cyclic AMP action, changing the natural concentrations of the substances for which it activates the modified gene which, in turn, brings the values to an optimal level. For example, should one wish for the beta cells of the pancreas to produce insulin, it is sufficient to lower blood glucose concentrations.

The vehicle according to the present invention is effective also in the case of hereditary diseases related to DNA repair means. In this case, the inserted gene will have to act also as a mold to repair the DNA hereditary disease before being in turn introduced into said DNA.

In particular, partly using the same method of the prior art, the desired result of accessing the nucleus is obtained without risking the above-cited disadvantages of the introduction of viral fragments.

Moreover, the vehicle of the invention activates a repair mechanism which allows to treat also gonadic cells, thereby avoiding the propagation of hereditary diseases to the offspring.

Advantageously, the vehicle according to the present invention may be employed in the foetus, through the administration of such vehicle to the mother during pregnancy. Thereby, treatment of a variety of diseases is obtained, even in a pre-natal stage, which may be useful in a number of genetic diseases.

Finally, the vehicle according to the present invention may also be employed for the specific introduction of damaging molecules, such as DDT for example, for the removal of malformed cells. This particular embodiment allows new therapies against tumours, hereditary diseases, for the removal of infected or ageing cells, or of cells loaded with accumulated substances, which may be due to environmental factors, but also to endogenously-altered metabolism production.

## Claims

1. Vehicle for gene therapy, **characterised in that** it consists of a molecule capable of binding with cyclic AMP.

2. Vehicle as claimed in claim 1), **characterised in that** said molecule is a steroid hormone.

3. Vehicle as claimed in claim 1) or 2), **characterised in that** said molecule is a sexual hormone.

4. Vehicle as claimed in any one of the preceding claims, **characterised in that** said molecule is a synthetic molecule.

5. Vehicle as claimed in claim 4), **characterised in that** said synthetic molecule is DDT.

6. Vehicle as claimed in anyone of the preceding claims, **characterised in that** it is used as a solution for parenteral administration.

7. Vehicle as claimed in any one of the preceding claims for use in a gene therapy, wherein gene arrangement on the chromosome occurs using the complex hormone-cyclic AMP-gene introduced in the strand as a mold for the DNA-repairing means.

8. Vehicle as claimed in any one of the preceding claims for use in a gene therapy, wherein gene activation occurs by changing the natural concentrations of the substances for which cyclic AMP activates the altered gene.

9. Vehicle as claimed in any one of the preceding claims for pre-natal therapies.

10. Vehicle as claimed in claim 9), **characterised in that** it is administered to a pregnant woman.

11. Vehicle as claimed in any one of the preceding claims, **characterised in that** it is capable of transporting damaging substances, for the removal of damaged cells.

12. Vehicle as claimed in claim 11), **characterised in that** said damaged cells have been affected by tumours, hereditary diseases, infection, ageing or accumulated substances.
